# EUROPEAN PATENT SPECIFICATION

(11) **EP 3 277 225 B1**
(45) Date of publication and mention of the grant of the patent: **18.01.2023**
(21) Application number: 15794773.0
(22) Date of filing: 05.11.2015
(51) Int. Cl.: A61F 2/30, A61B 17/04, A61B 17/84

(54) **SYSTEM FOR REPAIRING CARTILAGE DEFECTS**
SYSTEM FÜR DIE REPARATUR VON KNORPELDEFEKTEN
SYSTÈME POUR LA RÉPARATION DE DÉFAUTS CARTILAGINEUX

(30) Priority: 02.04.2015 US 201514677080
(43) Date of publication of application: 07.02.2018
(73) Proprietor: Arthrex Inc, Naples, FL 34108-1945 (US)
(72) Inventor: BENEDICT, Robert, Fort Myers, Florida 33967 (US); ROLLER, Brandon, Naples, Florida 34109 (US); SCHMIEDING, Reinhold, Naples, Florida 34108 (US); STOLL, Marc, Fort Myers, Florida 33908 (US)
(74) Representative: Marks & Clerk LLP
(86) International application number: PCT/US2015/059192
(87) International publication number: WO 2016/160068

(56) References cited:
- US-A1- 2004 093 031
- US-A1- 2008 153 157
- US-A1- 2008 255 613
- US-A1- 2008 281 422
- US-A1- 2010 168 869
- US-A1- 2013 338 792
- US-A1- 2015 045 768

## Description

### BACKGROUND

This disclosure relates to a system for knotlessly fixating a cartilage graft to bone to repair a cartilage defect.

Repetitive trauma to a joint, such as a knee, ankle, hip or shoulder joint, may cause cartilage defects. Cartilage defects include localized areas of damaged articular cartilage and, potentially, adjacent subchondral bone. Cartilage defects typically do not heal without treatment. If not treated, the defect could further deteriorate the articulate cartilage and/or underlying bone of the joint, thereby causing relatively significant arthritic pain in some individuals.

US 2008/281422 A1 (Schmieding) describes an arthroscopic method of resurfacing with a collagen patch. A collagen patch is prepared by dimensioning the patch to correspond to the articular surface, and by providing a series of loops installed along the edge of the patch. The loops correspond to holes on the articular surface. Once the patch has been successfully placed on the surface, the loops are positioned in close proximity to the pre-drilled holes. Fixation devices such as anchors may be installed over the loops, to attach the perimeter of the graft to the surface through the pre-drilled holes.

US 2004/093031 A1 (Burkhart et al) describes a method for securing soft tissue to bone which does not require the surgeon to tie suture knots to secure the tissue to the bone. This document discloses an eyelet implant at the distal end of a driver that securely engages and locks into a cannulated ribbed body of an interference plug or screw. The eyelet implant includes a fixed aperture for receiving a suture attached to a graft, such that the suture is able to freely slide through the aperture.

US 2010/0168869 A1 (Long et al) describes a tissue defect repair implant including a porous material, wherein the material is expandable or compressible. The implant may be a scaffold, and may also be formed of a material such as, for example, collagen or demineralized bone matrix.

US 2008/0255613 A1 (Kaiser et al) describes methods of attaching a soft tissue to an adjacent bone at a defect site. An adjustable loop region of a flexible construct contained in a bore defined by a fastener is passed through a tissue. The adjustable loop is passed through the tissue. The fastener is passed back through the adjustable loop to fold the adjustable loop upon itself. The fastener is attached to the bone.

US 2013/338792 A1 (Schmieding Reinhold et al) describes techniques, mixtures, mixing and delivery kits, and delivery instruments for implantation of micronized allograft tissue over a microfractured defect. Allograft cartilage tissue is delivered over a cartilage defect that has been debrided and microfractured, without the need for a periosteal covering or separate type of patch sewn over the top. The allograft tissue may be any micronized cartilage particulates obtained by various methods, for example, cartilage delivered in its native form, dehydrated via lyophilization, "freeze-dried," dehydrated via desiccation, or dehydrated by any other method.

### SUMMARY

Aspects of the present invention are set out in the appended claims. A method for repairing a cartilage defect according to an exemplary aspect of the present disclosure includes, among other things, preparing a cartilage defect for implantation of a cartilage graft and attaching the cartilage graft to bone using at least one knotless suture anchor.

In a further non-limiting embodiment of the foregoing method, the preparing step includes creating vertical margins around a periphery of the cartilage defect.

In a further non-limiting embodiment of either of the foregoing methods, the preparing step includes removing at least a portion of the cartilage defect using a curette.

In a further non-limiting embodiment of any of the foregoing methods, the preparing step includes performing bone marrow stimulation to the cartilage defect.

In a further non-limiting embodiment of any of the foregoing methods, the step of performing the bone marrow stimulation includes performing a microfracture procedure.

In a further non-limiting embodiment of any of the foregoing methods, the preparing step includes drying the cartilage defect.

In a further non-limiting embodiment of any of the foregoing methods, the attaching step includes passing a flexible strand through the cartilage graft, loading a free end of the flexible strand through a portion of the at least one knotless suture anchor, tensioning the flexible strand to approximate the cartilage graft to the bone and inserting the at least one knotless suture anchor into the bone to knotlessly fixate the cartilage graft to the bone.

In a further non-limiting embodiment of any of the foregoing methods, the inserting step includes moving an anchor body of the at least one knotless suture anchor toward the portion inside the bone to trap the flexible strand between the bone and the anchor body.

In a further non-limiting embodiment of any of the foregoing methods, the attaching step includes implanting the at least one knotless suture anchor into the bone, passing a flexible strand of the at least one knotless suture anchor through the cartilage graft and tensioning the flexible strand to approximate the cartilage graft to the bone.

In a further non-limiting embodiment of any of the foregoing methods, the tensioning step includes shuttling a free end of the flexible strand through the flexible strand to create a spliced loop around the cartilage graft.

In a further non-limiting embodiment of any of the foregoing methods, the at least one knotless suture anchor includes a first knotless suture anchor and a second knotless suture anchor. The attaching step includes implanting the first knotless suture anchor into the bone, passing a flexible strand connected to the first knotless suture anchor through the cartilage graft and tensioning the flexible strand to approximate the cartilage graft to the bone.

In a further non-limiting embodiment of any of the foregoing methods, the attaching step includes passing a second flexible strand through the cartilage graft, loading the second flexible strand through a portion of the second knotless suture anchor, tensioning the second flexible strand and inserting the second knotless suture anchor into bone.

In a further non-limiting embodiment of any of the foregoing methods, at least one of the first knotless suture anchor and the second knotless suture anchor is a soft knotless anchor assembly.

In a further non-limiting embodiment of any of the foregoing methods, at least one of the first knotless suture anchor and the second knotless suture anchor includes an eyelet.

In a further non-limiting embodiment of any of the foregoing methods, at least one of the first knotless suture anchor and the second knotless suture anchor includes a shuttle device configured to shuttle the flexible strand.

A method for repairing a cartilage defect according to another exemplary aspect of the present disclosure includes, among other things, passing a flexible strand through a cartilage graft, tensioning the flexible strand to approximate the cartilage graft relative to bone associated with the cartilage defect and inserting a knotless suture anchor into the bone to knotlessly fixate the cartilage graft to the bone.

In a further non-limiting embodiment of the foregoing method, the method includes creating a pilot hole in the bone prior to the step of inserting the knotless suture anchor.

In a further non-limiting embodiment of either of the foregoing methods, the tensioning step occurs before the inserting step.

In a further non-limiting embodiment of any of the foregoing methods, the tensioning step occurs after the inserting step.

In a further non-limiting embodiment of any of the foregoing methods, the method includes loading a free end of the flexible strand through a portion of the knotless suture anchor.

The embodiments, examples and alternatives of the preceding paragraphs, the claims, or the following description and drawings, including any of their various aspects or respective individual features, may be taken independently or in any combination. Features described in connection with one embodiment are applicable to all embodiments, unless such features are incompatible.

Accordingly, a method for repairing a cartilage defect according to an exemplary aspect of the present disclosure includes preparing a cartilage defect for implantation of a cartilage graft and attaching the cartilage graft to bone using at least one knotless suture anchor.

The preparing step may include one or more of creating vertical margins around a periphery of the cartilage defect, removing at least a portion of the cartilage defect using a curette, performing bone marrow stimulation to the cartilage defect and drying the cartilage defect. The step of performing the bone marrow stimulation may include performing a microfracture procedure.

The attaching step may include passing a flexible strand through the cartilage graft, loading a free end of the flexible strand through a portion of the at least one knotless suture anchor, tensioning the flexible strand to approximate the cartilage graft to the bone and inserting the at least one knotless suture anchor into the bone to knotlessly fixate the cartilage graft to the bone. The inserting step may include moving an anchor body of the at least one knotless suture anchor toward the portion inside the bone to trap the flexible strand between the bone and the anchor body. The tensioning step may include shuttling a free end of the flexible strand through the flexible strand to create a spliced loop around the cartilage graft. For example, the method includes preparing the cartilage defect by creating vertical margins around a periphery of the cartilage defect, removing at least a portion of the cartilage defect using a curette, performing bone marrow stimulation to the cartilage defect and drying the cartilage defect, and attaching the cartilage graft to bone by passing a flexible strand through the cartilage graft, loading a free end of the flexible strand through a portion of the at least one knotless suture anchor, tensioning the flexible strand to approximate the cartilage graft to the bone and inserting the at least one knotless suture anchor into the bone to knotlessly fixate the cartilage graft to the bone.

The attaching step may include implanting the at least one knotless suture anchor into the bone, passing a flexible strand of the at least one knotless suture anchor through the cartilage graft and tensioning the flexible strand to approximate the cartilage graft to the bone. The tensioning step may include shuttling a free end of the flexible strand through the flexible strand to create a spliced loop around the cartilage graft. For example, the method may include preparing the cartilage defect by creating vertical margins around a periphery of the cartilage defect, removing at least a portion of the cartilage defect using a curette, performing bone marrow stimulation to the cartilage defect and drying the cartilage defect, and attaching the cartilage graft to bone by implanting the at least one knotless suture anchor into the bone, passing a flexible strand of the at least one knotless suture anchor through the cartilage graft and tensioning the flexible strand to approximate the cartilage graft to the bone.

In a method for repairing a cartilage defect having any one or more of these features, the at least one knotless suture anchor may include a first knotless suture anchor and a second knotless suture anchor.

The preparing step may include one or more of creating vertical margins around a periphery of the cartilage defect, removing at least a portion of the cartilage defect using a curette, performing bone marrow stimulation to the cartilage defect and drying the cartilage defect. The step of performing the bone marrow stimulation may include performing a microfracture procedure.

The attaching step may include implanting the first knotless suture anchor into the bone, passing a flexible strand connected to the first knotless suture anchor through the cartilage graft and tensioning the flexible strand to approximate the cartilage graft to the bone. The attaching step may include passing a second flexible strand through the cartilage graft, loading the second flexible strand through a portion of the second knotless suture anchor, tensioning the second flexible strand and inserting the second knotless suture anchor into bone. The attaching step may include implanting the first knotless suture anchor into the bone, passing a flexible strand connected to the first knotless suture anchor through the cartilage graft and tensioning the flexible strand to approximate the cartilage graft to the bone and passing a second flexible strand through the cartilage graft, loading the second flexible strand through a portion of the second knotless suture anchor, tensioning the second flexible strand and inserting the second knotless suture anchor into bone. For example, the method may include preparing the cartilage defect by creating vertical margins around a periphery of the cartilage defect, removing at least a portion of the cartilage defect using a curette, performing bone marrow stimulation to the cartilage defect and drying the cartilage defect, and attaching the cartilage graft to bone by passing a flexible strand connected to the first knotless suture anchor through the cartilage graft and tensioning the flexible strand to approximate the cartilage graft to the bone and/or passing a second flexible strand through the cartilage graft, loading the second flexible strand through a portion of the second knotless suture anchor, tensioning the second flexible strand and inserting the second knotless suture anchor into bone.

In a method for repairing a cartilage defect having any one or more of the above features, at least one of the first knotless suture anchor and the second knotless suture anchor may be a soft knotless anchor assembly, and at least one of the first knotless suture anchor and the second knotless suture anchor may include one or more of an eyelet and a shuttle device configured to shuttle the flexible strand.

A method for repairing a cartilage defect according to another exemplary aspect of the present disclosure includes passing a flexible strand through a cartilage graft, tensioning the flexible strand to approximate the cartilage graft relative to bone associated with the cartilage defect and inserting a knotless suture anchor into the bone to knotlessly fixate the cartilage graft to the bone.

The tensioning step may occur before the inserting step, or the tensioning step may occur after the inserting step. The inserting step may include moving an anchor body of the knotless suture anchor toward the portion inside the bone to trap the flexible strand between the bone and the anchor body. The tensioning step may include shuttling a free end of the flexible strand through the flexible strand to create a spliced loop around the cartilage graft.

In a method for repairing a cartilage defect having any one or more of these features, the method may include one or more of creating a pilot hole in the bone prior to the step of inserting the knotless suture anchor and loading a free end of the flexible strand through a portion of the knotless suture anchor. For example, the method includes passing a flexible strand through a cartilage graft, tensioning the flexible strand to approximate the cartilage graft relative to bone associated with the cartilage defect, inserting a knotless suture anchor into the bone to knotlessly fixate the cartilage graft to the bone, where the tensioning step may occur before or after the inserting step and where a free end of the flexible strand may be loaded through a portion of the knotless suture anchor, and creating a pilot hole in the bone prior to the step of inserting the knotless suture anchor.

Aspects, features and embodiments of the present disclosure are described further below.

Generally, surgical methods for repairing cartilage defects of the present disclosure include attaching a cartilage graft to bone using at least one knotless suture anchor. The knotless suture anchor may be implanted into bone before tensioning a flexible strand, such as a suture, to approximate the cartilage graft to the bone. The knotless suture anchor may be implanted into bone after tensioning the flexible strand to approximate the cartilage graft to the bone.

A method of repairing a cartilage defect, such as a cartilage defect located within a joint, may be an arthroscopic method; however, the method could alternatively be performed as an open procedure. The cartilage defect may include osteochondral and/or chondral defects. In other words, the cartilage defect may include localized areas of damaged articular cartridge and/or damaged subchondral bone of the joint. The joint may be a knee joint. However, the methods of this disclosure may be used to repair cartilage defects located anywhere within the human body.

The cartilage graft may be knotlessly implanted after adequately preparing the cartilage defect. For example, an exemplary repair method may begin by prepping the cartilage defect for receiving a cartilage graft. After the surgeon has identified the cartilage defect within the joint, the cartilage defect may be debrided to a stable border having perpendicular margins. Tools, such as a curette and an elevator, can be used to create vertical margins around a periphery of the cartilage defect. The cartilage defect may be prepped with our without bone marrow stimulation. For example, the cartilage defect may be further prepped by performing bone marrow stimulation. For example, a microfracture procedure or some other technique may optionally be performed to obtain a bleeding bone bed. During the microfracture surgery, multiple perforations are created in subchondral bone that extends beneath the articular cartilage associated with the cartilage defect. The bleeding bone bed may be created using a tool, such as Arthrex's Powerpick^{™}, to create the perforations. Formation of the perforations creates the bleeding bone bed, which stimulates bone marrow seepage at the repair site. Other techniques can also be used to create the bleeding bone bed, including but not limited to, drilling, hammering, curetting, scraping, etc. The cartilage defect may also be dried to complete surgical preparation of the cartilage defect. The cartilage defect is dried to remove excess moisture that could interfere with implantation of the cartilage graft. The cartilage defect may be dried using any known technique.

The cartilage graft serves as a scaffold over the cartilage defect, thereby providing a tissue network that can potentially signal autologous cellular interactions. The size and shape of the cartilage graft may be selected using a template that is placed over the cartilage defect and marked to indicate its general size. The template may then be used to trim the cartilage graft down to the desired size and shape.

In an exemplary cartilage graft, the cartilage graft includes a cartilage disk having a plurality of pores formed through the cartilage disk. The cartilage graft may be made of human tissue (e.g., allograft cartilage), synthetic materials, xeno materials, etc. The cartilage graft may be made of a micronized cartilage matrix. The cartilage graft may be porous, but is not limited to such an embodiment.

For example, in an exemplary surgical method of the present disclosure for knotlessly attaching a cartilage graft to a bone using a knotless suture anchor, the cartilage graft may be secured to subchondral bone that is associated with the cartilage defect and which has been previously exposed during one or more steps for preparing the cartilage defect for implantation of the cartilage graft.

First, a flexible strand, such as a suture, is passed through the cartilage graft. A mattress stitch may be formed to connect the flexible strand to the cartilage graft. For example, the mattress stitch may be formed by inserting the flexible strand through a pore in a direction from the bottom toward a top of the cartilage disk of the cartilage graft and then inserting the flexible strand through an adjacent pore in a direction from the top toward the bottom of the disk. Other suturing techniques and configurations are also contemplated within the scope of this disclosure. For example, in situations where the cartilage graft is not porous, the flexible strand may be simply threaded through the cartilage graft.

Next, one or more free ends of the flexible strand are loaded through a portion of the knotless suture anchor. In one example, the portion includes an eyelet of the knotless suture anchor. The eyelet may next be inserted into the bone. The eyelet can be inserted into a pilot hole that is pre-formed in the bone or the eyelet itself can form the pilot hole. Once the eyelet is positioned at least partially in the pilot hole, the flexible strand may be tensioned in a direction to approximate the cartilage graft to the bone. In one example, tensioning the flexible strand positions the cartilage graft in place over the subchondral bone that is associated with the cartilage defect.

Finally, the knotless suture anchor is inserted into the bone to knotlessly fixate the cartilage graft to the bone. For example, an anchor body of the knotless suture anchor may be moved toward the eyelet to trap the flexible strand between the bone and the anchor body in order to fixate the cartilage graft in place. Free ends of the flexible strand may be trimmed flush to the cartilage graft. The procedure may be repeated to implant multiple knotless suture anchors to fixate the cartilage graft in place.

In another example, in a surgical method of the present disclosure for knotlessly fixating a cartilage graft to bone, a knotless suture anchor is first inserted into the bone. A pilot hole may optionally be pre-formed into the bone for receiving the knotless suture anchor. A flexible strand that is connected to the knotless suture anchor may next be passed through the cartilage graft. For example, the flexible strand may be looped through one or more pores of the cartilage graft. Finally, the flexible strand may be tensioned to approximate the cartilage graft to the bone. In one example, tensioning the flexible strand shuttles the flexible strand through the knotless suture anchor to tighten the cartilage graft down over the subchondral bone that is associated with the cartilage defect. The procedure may be repeated to implant multiple knotless suture anchors to fixate the cartilage graft in place.

The techniques of each of the exemplary surgical methods described above are considered "knotless" because there is no need to tie knots in the flexible strand in order to secure the cartilage graft to the bone.

In another example, the techniques of both of the exemplary surgical methods described above may be utilized to fixate the cartilage graft to the bone. That is, the technique in which the step of tensioning the flexible strand precedes the step of inserting the knotless suture anchor into the bone may be combined with the technique in which the step of inserting the knotless suture anchor into the bone precedes the step of tensioning the flexible strand. In other words, multiple knotless suture anchors may be utilized to fixate the cartilage graft, for example, including a combination of different types of knotless suture anchors to fixate the cartilage graft, for example.

Multiple different fixation patterns may be used to secure the cartilage graft to the bone. For example, a cartilage graft may be fixated by positioning flexible strands at each of its four quadrants, through its center and about its periphery, through its two halves, or at each third of the cartilage graft. Other fixation patterns may also be used.

In another example, a layer of fibrin may be applied over the cartilage graft after it has been fixated to bone. The fibrin may be applied using an applicator. After the fibrin and the cartilage graft sit for a predefined amount of time, such as approximately five minutes, the joint may be gently ranged before closure to assure adherence of the fibrin and the cartilage graft to the bone.

Various exemplary knotless suture anchors may be used in the cartilage defect repair methods described above. Knotless suture anchors may be utilized alone or in combination with one another to fixate a cartilage graft to bone.

For example, an exemplary knotless suture anchor includes an anchor body and an eyelet. The anchor body is pre-loaded onto a driver. The anchor body may be configured as a screw or an interference plug which are appropriately cannulated for receiving a shaft of the driver. The eyelet may be provided at a distal end of the driver. The eyelet is releasably attached to the distal end of the driver. The eyelet includes an aperture for receiving one or more flexible strands.

For example, another exemplary knotless suture anchor includes an anchor body and a flexible strand that extends inside of the anchor body. A shuttle device, such as a nitinol wire, is also received inside the anchor body. The shuttle device may be pre-assembled to the flexible strand and will form a spliced loop after the flexible strand is shuttled through itself. For example, a free end of the flexible strand is passed through eyelet of the shuttle device and then the shuttle device is pulled to allow the flexible strand to pass through itself and form the spliced loop. The perimeter of the spliced loop is adjustable to allow the construct to be self-cinching and to adjust the tension on the cartilage graft that is to be fixated.

For example, in yet another exemplary knotless suture anchor, the knotless suture anchor is a "soft" anchor assembly formed of soft materials such as yarns, fibers, filaments, strings, fibrils, strands, sutures, etc., or any combination of such materials. The soft materials may be synthetic or natural materials, or combinations of synthetic and natural materials, and may be bio-degradable or non-degradable within the scope of this disclosure. In one example, the knotless suture anchor is made exclusively of soft, suture-based materials.

Such a knotless suture anchor includes an anchor body and a flexible strand received through the anchor body. The flexible strand includes an eyelet (located at one end of the strand) that is preloaded with a shuttle device attached at the portion of the flexible strand that exits the anchor body (for example, at a portion of the other end of the strand). In one example, the shuttle device is a nitinol passing wire. The shuttle device may be pre-assembled to the flexible strand and will form a spliced loop after the flexible strand is shuttled through itself. For example, a free end of the flexible strand is passed through the eyelet of the shuttle device and then the shuttle device is pulled to allow the flexible strand to pass through itself and form the spliced loop. The perimeter of the spliced loop is adjustable to allow the construct to be self-cinching and to adjust the tension on the cartilage graft to be fixated.

As is apparent to those skilled in the art from the foregoing disclosure, while different non-limiting embodiments are illustrated as having specific components, the embodiments of this disclosure are not limited to those particular combinations. It is possible to use some of the components or features from any of the non-limiting embodiments in combination with features or components from any of the other non-limiting embodiments.

The various features and advantages of this disclosure will become apparent to those skilled in the art from the following detailed description. The drawings that accompany the detailed description can be briefly described as follows.

### BRIEF DESCRIPTION OF THE DRAWINGS

Figures 1 and 2 schematically illustrate preparing a cartilage defect for implantation of a cartilage graft.
Figure 3 illustrates an exemplary cartilage graft.
Figures 4A, 4B, 4C and 4D schematically illustrate knotlessly fixating a cartilage graft to bone according to a first embodiment of this disclosure.
Figures 5A, 5B and 5C schematically illustrate knotlessly fixating a cartilage graft to bone according to a second embodiment of this disclosure.
Figures 6A, 6B, 6C and 6D illustrate exemplary fixation patterns for fixating a cartilage graft to a bone.
Figure 7 illustrates applying a layer of fibrin over a repaired cartilage defect.
Figure 8 illustrates a knotless suture anchor according to a first embodiment of this disclosure.
Figures 9 and 10 illustrate a knotless suture anchor according to a second embodiment of this disclosure.
Figures 11 and 12 illustrate a knotless suture anchor according to yet another embodiment of this disclosure.

### DETAILED DESCRIPTION

This disclosure describes surgical methods for repairing cartilage defects. The surgical methods include attaching a cartilage graft to bone using at least one knotless suture anchor. In some embodiments, the knotless suture anchor is implanted into bone before tensioning a flexible strand, such as a suture, to approximate the cartilage graft to the bone. In other embodiments, the knotless suture anchor is implanted into bone after tensioning the flexible strand to approximate the cartilage graft to the bone. These and other features are described in greater detail in the following paragraphs of this detailed description.

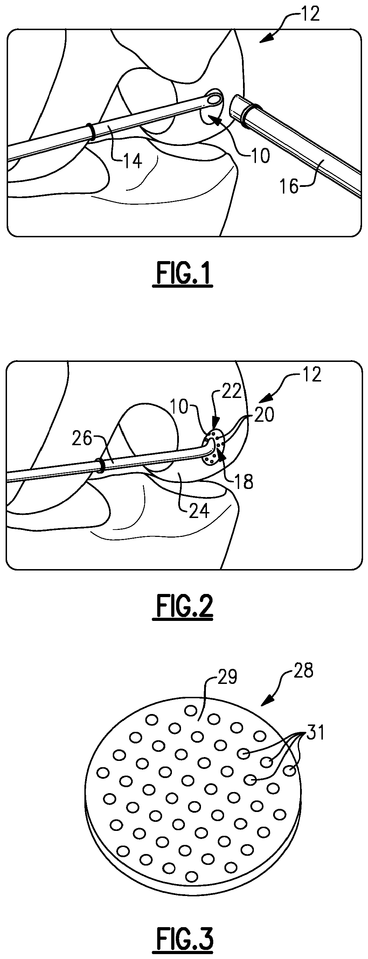

Figures 1-7 schematically illustrate a method of repairing a cartilage defect 10 located within a joint 12. The method is illustrated and described as an arthroscopic method; however, the method could alternatively be performed as an open procedure. The cartilage defect 10 can include osteochondral and/or chondral defects. In other words, the cartilage defect 10 may include localized areas of damaged articular cartridge and/or damaged subchondral bone of the joint 12. In one embodiment, the joint 12 is a knee joint. However, the methods of this disclosure may be used to repair cartilage defects located anywhere within the human body.

The exemplary repair method begins by prepping the cartilage defect 10 for receiving a cartilage graft 28 (shown in Figure 3). Referring first to Figure 1, after the surgeon has identified the cartilage defect 10 within the joint 12, the cartilage defect 10 may be debrided to a stable border having perpendicular margins. Tools, such as a curette 14 and an elevator 16, can be used to create vertical margins around a periphery of the cartilage defect 10.

The cartilage defect 10 may be prepped with our without bone marrow stimulation. In one non-limiting embodiment, the cartilage defect 10 is further prepped by performing bone marrow stimulation. For example, as shown in Figure 2, a microfracture procedure or some other technique may optionally be performed to obtain a bleeding bone bed 18. During the microfracture surgery, multiple perforations 20 are created in subchondral bone 22 that extends beneath the articular cartilage 24 associated with the cartilage defect 10. The bleeding bone bed 18 may be created using a tool 26, such as Arthrex's Powerpick^{™}, to create the perforations 20. Formation of the perforations 20 creates the bleeding bone bed 18, which stimulates bone marrow seepage at the repair site. Other techniques can also be used to create the bleeding bone bed 18, including but not limited to, drilling, hammering, curetting, scraping, etc.

The cartilage defect 10 may also be dried to complete surgical preparation of the cartilage defect 10. The cartilage defect 10 is dried to remove excess moisture that could interfere with implantation of the cartilage graft 28. The cartilage defect 10 may be dried using any known technique.

The cartilage graft 28 may be knotlessly implanted after adequately prepping the cartilage defect 10. The cartilage graft 28 serves as a scaffold over the cartilage defect 10, thereby providing a tissue network that can potentially signal autologous cellular interactions. The size and shape of the cartilage graft 28 may be selected using a template that is placed over the cartilage defect 10 and marked to indicate its general size. The template may then be used to trim to cartilage graft 28 down to the desired size and shape.

One exemplary cartilage graft 28 is illustrated in Figure 3. In one non-limiting embodiment, the cartilage graft 28 includes a cartilage disk 29 having a plurality of pores 31 formed through the cartilage disk 29. The cartilage graft 28 may be made of human tissue (e.g., allograft cartilage), synthetic materials, xeno materials, etc. In one non-limiting embodiment, the cartilage graft 28 is made of a micronized cartilage matrix. Although shown as being porous, the cartilage graft 28 is not limited to such an embodiment.

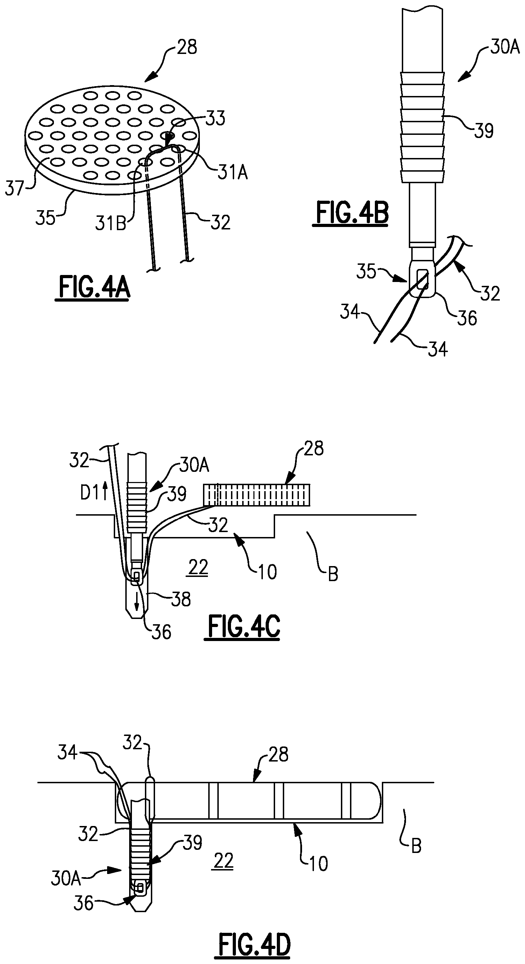

Figures 4A-4D illustrate a first non-limiting embodiment for knotlessly attaching the cartilage graft 28 to a bone B using a knotless suture anchor 30A. The cartilage graft 28 may be secured to subchondral bone 22 that is associated with the cartilage defect 10 and which has been previously exposed during one or more of the method steps shown in Figures 1 and 2.

First, as shown in Figure 4A, a flexible strand 32, such as a suture, is passed through the cartilage graft 28. A mattress stitch 33 may be formed to connect the flexible strand 32 to the cartilage graft 28. The mattress stitch 33 is formed by inserting the flexible strand 32 through a pore 31A in a direction from the bottom 35 toward a top 37 of the disk 29 of the cartilage graft 28 and then inserting the flexible strand 32 through an adjacent pore 31B in a direction from the top 37 toward the bottom 35 of the disk 29. Other suturing techniques and configurations are also contemplated within the scope of this disclosure. For example, in situations where the cartilage graft 28 is not porous, the flexible strand 32 may be simply threaded through the cartilage graft 28.

Next, as shown in Figure 4B, one or more free ends 34 of the flexible strand 32 are loaded through a portion 35 of the knotless suture anchor 30A. In one non-limiting embodiment, the portion 35 includes an eyelet 36 of the knotless suture anchor 30A. The eyelet 36 may next be inserted into the bone B, as shown in Figure 4C. The eyelet 36 can be inserted into a pilot hole 38 that is pre-formed in the bone B or the eyelet 36 itself can form the pilot hole 38. Once the eyelet 36 is positioned at least partially in the pilot hole 38, the flexible strand 32 may be tensioned in a direction D1 to approximate the cartilage graft 28 to the bone B. In one embodiment, tensioning the flexible strand 32 positions the cartilage graft 28 in place over the subchondral bone 22 that is associated with the cartilage defect 10.

Finally, as shown in Figure 4D, the knotless suture anchor 30A is inserted into the bone B to knotlessly fixate the cartilage graft 28 to the bone B. For example, an anchor body 39 of the knotless suture anchor 30A may be moved toward the eyelet 36 to trap the flexible strand 32 between the bone B and the anchor body 39 in order to fixate the cartilage graft 28 in place. Free ends 34 of the flexible strand 32 may be trimmed flush to the cartilage graft 28. The procedure illustrated in Figures 4A-4D can be repeated to implant multiple knotless suture anchors 30A to fixate the cartilage graft 28 in place.

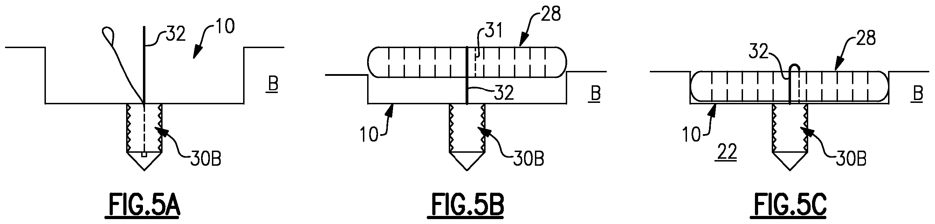

Figures 5A-5C illustrate another non-limiting embodiment for knotlessly fixating the cartilage graft 28 to bone B. In this embodiment, a knotless suture anchor 30B is first inserted into the bone B (see Figure 5A). A pilot hole 38 may optionally be pre-formed into the bone B for receiving the knotless suture anchor 30B. A flexible strand 32 that is connected to the knotless suture anchor 30 may next be passed through the cartilage graft 28 (see Figure 5B). For example, the flexible strand 32 may be looped through one or more pores 31 of the cartilage graft 28. Finally, as shown in Figure 5C, the flexible strand 32 may be tensioned to approximate the cartilage graft 28 to the bone B. In one embodiment, tensioning the flexible strand 32 shuttles the flexible strand 32 through the knotless suture anchor 30B to tighten the cartilage graft 28 down over the subchondral bone 22 that is associated with the cartilage defect 10. The procedure illustrated in Figures 5A-5C can be repeated to implant multiple knotless suture anchors 30B to fixate the cartilage graft 28 in place.

The techniques shown in Figures 4A-4D and 5A-5C are considered "knotless" because there is no need to tie knots in the flexible strand 32 in order to secure the cartilage graft 28 to the bone B. In another non-limiting embodiment, both the technique described by Figures 4A-4D and the technique described by Figures 5A-5C may be utilized to fixate the cartilage graft 28 to the bone B. In other words, a combination of different types of knotless suture anchors may be utilized to fixate the cartilage graft 28.

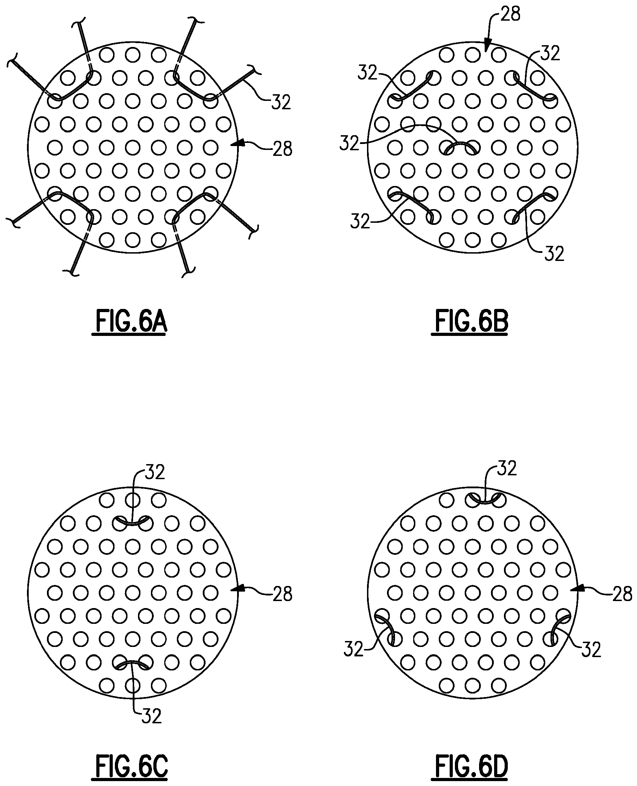

Multiple different fixation patterns may be used to secure the cartilage graft 28 to the bone B. For example, the cartilage graft 28 can be fixated by positioning flexible strands 32 at each of its four quadrants (see Figure 6A), through its center and about its periphery (see Figure 6B), through its top and bottom halves (see Figure 6C), or at each third of the cartilage graft 28 (see Figure 6D). Other fixation patterns could also be used.

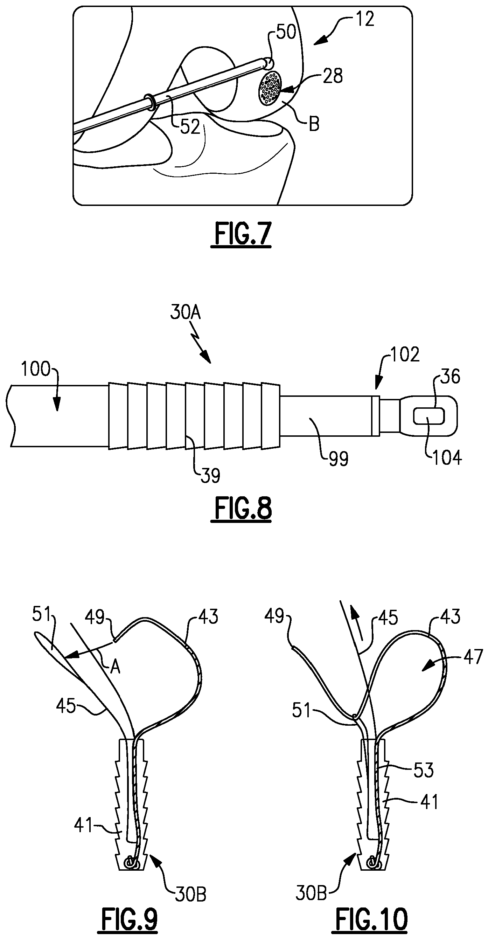

In another embodiment, as shown in Figure 7, a layer of fibrin 50 may be applied over the cartilage graft 28 after it has been fixated to bone B. The fibrin 50 may be applied using an applicator 52. After the fibrin 50 and the cartilage graft 28 sit for a predefined amount of time, such as approximately five minutes, the joint 12 may be gently ranged before closure to assure adherence of the fibrin 50 and the cartilage graft 28 to the bone B.

Figures 8-12 illustrate exemplary knotless suture anchors that can be used in the cartilage defect repair methods described above. Knotless suture anchors similar to those shown in Figures 8-12 may be utilized alone or in combination with one another to fixate a cartilage graft to bone.

Referring first to Figure 8, the knotless suture anchor 30A includes an anchor body 39 and an eyelet 36. The anchor body 39 is pre-loaded onto a driver 100. The anchor body 39 may be configured as a screw or an interference plug which are appropriately cannulated for receiving a shaft 99 of the driver 100. The eyelet 36 may be provided at a distal end 102 of driver 100. The eyelet 36 is releasably attached to the distal end 102 of driver 100. The eyelet 36 includes an aperture 104 for receiving one or more flexible strands (see, for example, the flexible strand 32 shown in Figure 4B).

Figures 9-10 illustrate another knotless suture anchor 30B. In this embodiment, the knotless suture anchor 30B includes an anchor body 41 and a flexible strand 43 that extends inside of the anchor body 41. A shuttle device 45, such as a nitinol wire, is also received inside the anchor body 41. The shuttle device 45 may be pre-assembled to the flexible strand 43 as shown in Figure 9 and will form a spliced loop 47 as shown in Figure 10 after the flexible strand 43 is shuttled through itself. For example, a free end 49 of flexible strand 43 is passed through eyelet 51 of the shuttle device 45 (in the direction of arrow A of Figure 9) and then the shuttle device 45 is pulled to allow flexible strand 43 to pass through itself at region 53 (see Figure 10) and form the spliced loop 47. The perimeter of spliced loop 47 is adjustable to allow the construct to be self-cinching and to adjust the tension on the cartilage graft that is to be fixated.

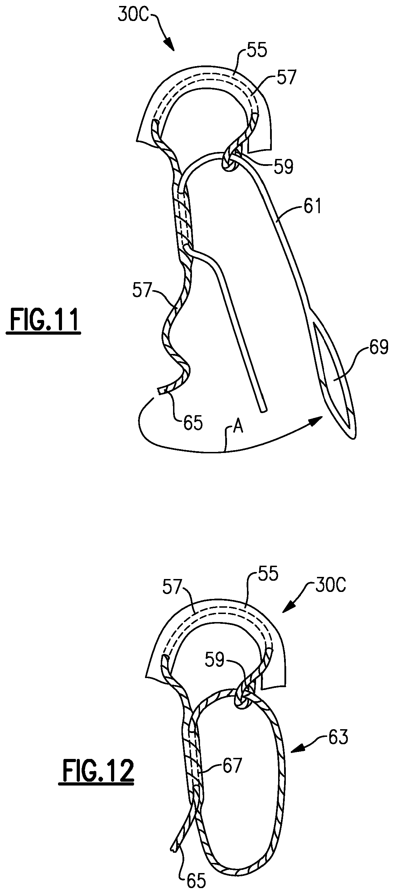

Figures 11-12 illustrates yet another knotless suture anchor 30C. In this embodiment, the knotless suture anchor 30C is a "soft" anchor assembly formed of soft materials such as yarns, fibers, filaments, strings, fibrils, strands, sutures, etc., or any combination of such materials. The soft materials may be synthetic or natural materials, or combinations of synthetic and natural materials, and may be bio-degradable or non-degradable within the scope of this disclosure. In one non-limiting embodiment, the knotless suture anchor 30C is made exclusively of soft, suture-based materials.

The knotless suture anchor 30C includes an anchor body 55 and a flexible strand 57 received through the anchor body 55. The flexible strand 57 includes an eyelet 59 (located at one end of the strand) that is preloaded with a shuttle device 61 attached at the portion of the flexible strand 57 that exits the anchor body 55 (for example, at a portion of the other end of the strand). In one embodiment, the shuttle device 61 is a nitinol passing wire. The shuttle device 61 may be pre-assembled to the flexible strand 57 as shown in Figure 11 and will form a spliced loop 63 as shown in Figure 12 after the flexible strand 57 is shuttled through itself. For example, a free end 65 of flexible strand 57 is passed through eyelet 69 of the shuttle device 61 (in the direction of arrow A of Figure 11) and then the shuttle device 61 is pulled to allow flexible strand 57 to pass through itself at region 67 (see Figure 12) and form the spliced loop 63. The perimeter of spliced loop 63 is adjustable to allow the construct to be self-cinching and to adjust the tension on the cartilage graft to be fixated.

Although the different non-limiting embodiments are illustrated as having specific components, the embodiments of this disclosure are not limited to those particular combinations. It is possible to use some of the components or features from any of the non-limiting embodiments in combination with features or components from any of the other non-limiting embodiments.

It should be understood that like reference numerals identify corresponding or similar elements throughout the several drawings. It should also be understood that although a particular component arrangement is disclosed and illustrated in these exemplary embodiments, other arrangements could also benefit from the teachings of this disclosure.

The foregoing description shall be interpreted as illustrative and not in any limiting sense. A worker of ordinary skill in the art would understand that certain modifications could come within the scope of this disclosure. For these reasons, the following claims should be studied to determine the true scope and content of this disclosure.

## Claims

1. A system for repairing a cartilage defect, comprising:
a cartilage graft (28) configured for repairing the cartilage defect (10),
wherein the cartilage graft (28) includes a cartilage disk (29) that includes a plurality of pores (31) formed through the cartilage disk (29); and
a flexible strand (32, 43, 57) passed through a pore (31A) and an adjacent pore (31B) of the plurality of pores (31);
**characterized in that** the cartilage graft (28) is made of micronized cartilage matrix and the system further comprises at least one knotless suture anchor (30A, 30B, 30C) configured for attaching the cartilage graft (28) to a bone.

2. The system as recited in claim 1, comprising a curette (14) configured for removing at least a portion of the cartilage defect (10).

3. The system as recited in claim 1, comprising a tool (26) configured for performing bone marrow stimulation to the cartilage defect.

4. The system as recited in claim 3, wherein the tool (26) is configured to create a plurality of perforations in the bone.

5. The system as recited in claim 1, wherein:
a free end (34) of the flexible strand (32) is loaded through a portion (35) of the at least one knotless suture anchor (30A); and
wherein the flexible strand (32) is tensionable to approximate the cartilage graft (28) to the bone.

6. The system as recited in claim 5, wherein the at least one knotless suture anchor (30A) includes an anchor body (39) movable toward a portion (35) to trap the flexible strand (32) between the bone and the anchor body (39).

7. The system as recited in claim 1, wherein:
the flexible strand (32, 43, 57) is tensionable to approximate the cartilage graft to the bone.

8. The system as recited in claim 7, wherein a free end (34, 65) of the flexible strand (43, 57) passes through the flexible strand (43, 57) to establish a spliced loop (47, 63) around the cartilage graft (28).

9. The system as recited in claim 1, wherein the at least one knotless suture anchor (30A, 30B, 30C) includes a first knotless suture anchor (30A, 30B, 30C) and a second knotless suture anchor (30A, 30B, 30C)

10. The system as recited in claim 9, comprising:
a first flexible strand (32, 43, 57) loaded through a portion of the first knotless suture anchor (30A, 30B, 30C);
a second flexible strand (32, 43, 57) loaded through a portion of the second knotless suture anchor (30A, 30B, 30C).

11. The system as recited in claim 9 or 10, wherein at least one of the first knotless suture anchor (30C) and the second knotless suture anchor (30C) is a soft knotless anchor assembly made exclusively of soft, suture-based materials.

12. The system as recited in any of claims 9, 10 or 11, wherein at least one of the first knotless suture anchor (30A) and the second knotless suture anchor (30A) includes an eyelet (59).

13. The system as recited in any of claims 9 to 12, wherein at least one of the first knotless suture anchor (30B, 30C) and the second knotless suture anchor (30B, 30C) includes a shuttle device (45, 61) configured to shuttle a flexible strand (43, 57).

14. The system as recited in any preceding claim, wherein the flexible strand (32, 43, 57) forms a mattress stitch (33) to connect the flexible strand (32, 43, 57) to the cartilage graft (28).

15. The system as recited in claim 14, wherein the mattress stitch (33) is formed by inserting the flexible strand (32, 43, 57) through the pore (31A) in a direction from a bottom (35) toward a top (37) of the cartilage disk (29) and then inserting the flexible strand (32, 43, 57) through the adjacent pore (31B) in a direction from the top (37) toward the bottom (35) of the cartilage disk (29).

16. The system as recited in claim 13, wherein the shuttle device (45, 61) is a nitinol wire.

## Patentansprüche

1. System für die Reparatur von Knorpeldefekten, umfassend:
ein zum Reparieren des Knorpeldefekts (10) ausgelegtes Knorpeltransplantat (28),
wobei das Knorpeltransplantat (28) eine Knorpelscheibe (29) aufweist, die eine Vielzahl von durch die Knorpelscheibe (29) hindurch ausgebildeten Poren (31) aufweist; und
einen durch eine Pore (31A) und eine benachbarte Pore (31B) der Vielzahl von Poren (31) geführten flexiblen Strang (32, 43, 57) ;
**dadurch gekennzeichnet, dass** das Knorpeltransplantat (28) aus mikronisierter Knorpelmatrix hergestellt ist und das System ferner zumindest einen knotenlosen Nahtanker (30A, 30B, 30C) umfasst, der zur Befestigung des Knorpeltransplantats (28) an einem Knochen ausgebildet ist.

2. System nach Anspruch 1, umfassend eine zur Entfernung zumindest eines Teils des Knorpeldefekts (10) ausgebildete Kürette (14).

3. System nach Anspruch 1, umfassend ein zur Durchführung einer Knochenmarkstimulation an dem Knorpeldefekt ausgebildetes Werkzeug (26).

4. System nach Anspruch 3, wobei das Werkzeug (26) zur Erzeugung einer Vielzahl von Perforationen in dem Knochen ausgebildet ist.

5. System nach Anspruch 1, wobei:
ein freies Ende (34) des flexiblen Strangs (32) durch einen Abschnitt (35) des zumindest einen knotenlosen Nahtankers (30A) eingeführt ist; und
wobei der flexible Strang (32) zur Annäherung des Knorpeltransplantats (28) an den Knochen spannbar ist.

6. System nach Anspruch 5, wobei der zumindest eine knotenlose Nahtanker (30A) einen in Richtung eines Abschnitts (35) beweglichen Ankerkörper (39) aufweist, um den flexiblen Strang (32) zwischen dem Knochen und dem Ankerkörper (39) festzuhalten.

7. System nach Anspruch 1, wobei:
wobei der flexible Strang (32, 43, 57) zur Annäherung des Knorpeltransplantats an den Knochen spannbar ist.

8. System nach Anspruch 7, wobei ein freies Ende (34, 65) des flexiblen Strangs (43, 57) durch den flexiblen Strang (43, 57) verläuft, um eine gespleißte Schlaufe (47, 63) um das Knorpeltransplantat (28) zu bilden.

9. System nach Anspruch 1, wobei der zumindest eine knotenlose Nahtanker (30A, 30B, 30C) einen ersten knotenlosen Nahtanker (30A, 30B, 30C) und einen zweiten knotenlosen Nahtanker (30A, 30B, 30C) aufweist.

10. System nach Anspruch 9, umfassend:
einen ersten flexiblen Strang (32, 43, 57), der durch einen Abschnitt des ersten knotenlosen Nahtankers (30A, 30B, 30C) eingeführt ist;
einen zweiten flexiblen Strang (32, 43, 57), der durch einen Abschnitt des zweiten knotenlosen Nahtankers (30A, 30B, 30C) eingeführt ist.

11. System nach Anspruch 9 oder 10, wobei zumindest einer von dem ersten knotenlosen Nahtanker (30C) und dem zweiten knotenlosen Nahtanker (30C) eine weiche, knotenlose Ankeranordnung ist, die ausschließlich aus weichen, auf Nahtmaterial basierenden Materialien hergestellt ist.

12. System nach einem der Ansprüche 9, 10 oder 11, wobei zumindest einer von dem ersten knotenlosen Nahtanker (30A) und dem zweiten knotenlosen Nahtanker (30A) eine Öse (59) aufweist.

13. System nach einem der Ansprüche 9 bis 12, wobei zumindest einer des ersten knotenlosen Nahtankers (30B, 30C) und des zweiten knotenlosen Nahtankers (30B, 30C) eine Shuttle-Vorrichtung (45, 61) aufweist, die für die Hin- und Herbewegung eines flexiblen Strangs (43, 57) ausgebildet ist.

14. System nach einem der vorhergehenden Ansprüche, wobei der flexible Strang (32, 43, 57) einen Matratzenstich (33) bildet, um den flexiblen Strang (32, 43, 57) mit dem Knorpeltransplantat (28) zu verbinden.

15. System nach Anspruch 14, wobei der Matratzenstich (33) durch Einführen des flexiblen Strangs (32, 43, 57) durch die Pore (31A) in einer Richtung von einer Unterseite (35) zu einer Oberseite (37) der Knorpelscheibe (29) und anschließendes Einführen des flexiblen Strangs (32, 43, 57) durch die benachbarte Pore (31B) in einer Richtung von der Oberseite (37) zu der Unterseite (35) der Knorpelscheibe (29) gebildet wird.

16. System nach Anspruch 13, wobei die Shuttle-Vorrichtung (45, 61) ein Nitinoldraht ist.

## Revendications

1. Système pour la réparation d'un défaut de cartilage, comprenant :
une greffe de cartilage (28) configurée pour la réparation du défaut de cartilage (10),
dans lequel la greffe de cartilage (28) comprend un disque de cartilage (29) qui comprend une pluralité de pores (31) formés à travers le disque de cartilage (29) ; et
un brin flexible (32, 43, 57) passé à travers un pore (31A) et un pore adjacent (31B) parmi la pluralité de pores (31) ;
**caractérisé en ce que** la greffe de cartilage (28) est constituée d'une matrice de cartilage micronisée et le système comprend en outre au moins un ancrage de suture sans nœud (30A, 30B, 30C) configuré pour la fixation de la greffe de cartilage (28) à un os.

2. Système selon la revendication 1, comprenant une curette (14) configurée pour l'enlèvement d'au moins une partie du défaut de cartilage (10).

3. Système selon la revendication 1, comprenant un outil (26) configuré pour la réalisation d'une stimulation de la moelle osseuse sur le défaut de cartilage.

4. Système selon la revendication 3, dans lequel l'outil (26) est configuré pour créer une pluralité de perforations dans l'os.

5. Système selon la revendication 1, dans lequel :
une extrémité libre (34) du brin flexible (32) est chargée à travers une partie (35) de l'au moins un ancrage de suture sans nœud (30A) ; et
dans lequel le brin flexible (32) peut être mis sous tension pour rapprocher la greffe de cartilage (28) de l'os.

6. Système selon la revendication 5, dans lequel l'au moins un ancrage de suture sans nœud (30A) comprend un corps d'ancrage (39) mobile vers une partie (35) pour piéger le brin flexible (32) entre l'os et le corps d'ancrage (39).

7. Système selon la revendication 1, dans lequel :
le brin flexible (32, 43, 57) peut être mis sous tension pour rapprocher la greffe de cartilage de l'os.

8. Système selon la revendication 7, dans lequel une extrémité libre (34, 65) du brin flexible (43, 57) passe à travers le brin flexible (43, 57) pour établir une boucle épissée (47, 63) autour de la greffe de cartilage (28).

9. Système selon la revendication 1, dans lequel l'au moins un ancrage de suture sans nœud (30A, 30B, 30C) comprend un premier ancrage de suture sans nœud (30A, 30B, 30C) et un deuxième ancrage de suture sans nœud (30A, 30B, 30C)

10. Système selon la revendication 9, comprenant :
un premier brin flexible (32, 43, 57) chargé à travers une partie du premier ancrage de suture sans nœud (30A, 30B, 30C) ;
un deuxième brin flexible (32, 43, 57) chargé à travers une partie du deuxième ancrage de suture sans nœud (30A, 30B, 30C).

11. Système selon la revendication 9 ou 10, dans lequel au moins un parmi le premier ancrage de suture sans nœud (30C) et le deuxième ancrage de suture sans nœud (30C) est un ensemble d'ancrage sans nœud souple réalisé exclusivement en matériaux à base de suture, souples.

12. Système selon l'une quelconque des revendications 9, 10 ou 11, dans lequel au moins un parmi le premier ancrage de suture sans nœud (30A) et le deuxième ancrage de suture sans nœud (30A) comprend un œillet (59).

13. Système selon l'une quelconque des revendications 9 à 12, dans lequel au moins un parmi le premier ancrage de suture sans nœud (30B, 30C) et le deuxième ancrage de suture sans nœud (30B, 30C) comprend un dispositif de navette (45, 61) configuré pour navetter un brin flexible (43, 57).

14. Système selon l'une quelconque des revendications précédentes, dans lequel le brin flexible (32, 43, 57) forme un point de matelassier (33) pour relier le brin flexible (32, 43, 57) à la greffe de cartilage (28).

15. Système selon la revendication 14, dans lequel le point de matelassier (33) est formé par l'insertion du brin flexible (32, 43, 57) à travers le pore (31A) dans une direction à partir d'un fond (35) vers un sommet (37) du disque de cartilage (29) et ensuite l'insertion du brin flexible (32, 43, 57) à travers le pore adjacent (31B) dans une direction à partir du sommet (37) vers le fond (35) du disque de cartilage (29).

16. Système selon la revendication 13, dans lequel le dispositif de navette (45, 61) est un fil de nitinol.
